# EUROPÄISCHE PATENTSCHRIFT

(11) **EP 2 225 560 B1**
(45) Veröffentlichungstag und Bekanntmachung des Hinweises auf die Patenterteilung: **28.06.2017**
(21) Anmeldenummer: 08853543.0
(22) Anmeldetag: 25.11.2008
(51) Int. Cl.: G01N 33/53, G01N 33/531, C08G 63/91

(54) **AFFINITÄTSSTRUKTUREN ZUR SPEZIFISCHEN BINDUNG VON SUBSTANZEN DURCH NICHT-KOVALENTE INTERAKTIONSFORMEN**
AFFINITY STRUCTURES FOR THE SPECIFIC BINDING OF SUBSTANCES BY MEANS OF NON-COVALENT INTERACTION SHAPES
STRUCTURES D'AFFINITÉ POUR LA LIAISON SPÉCIFIQUE DE SUBSTANCES PAR DES FORMES D'INTERACTION NON COVALENTES

(30) Priorität: 26.11.2007 DE 102007056875
(43) Veröffentlichungstag der Anmeldung: 08.09.2010
(73) Patentinhaber: Fraunhofer-Gesellschaft zur Förderung der angewandten Forschung e.V., 80686 München (DE)
(72) Erfinder: ANDRESEN, Heiko, Hitchin SG4 9SD (GB); BIER, Frank, 14482 Potsdam (DE); KATTERLE, Martin, 14612 Falkensee (DE)
(74) Vertreter: Katzameyer, Michael
(86) Internationale Anmeldenummer: PCT/EP2008/009984
(87) Internationale Veröffentlichungsnummer: WO 2009/068244

(56) Entgegenhaltungen:
- US-A1- 2006 240 435
- CHOU P C ET AL: "C-reactive protein thin-film molecularly imprinted polymers formed using a micro-contact approach" ANALYTICA CHIMICA ACTA, ELSEVIER, AMSTERDAM, NL, Bd. 542, Nr. 1, 22. Juni 2005 (2005-06-22), Seiten 20-25, XP004913171 ISSN: 0003-2670
- HOLTHOFF ET AL: "Molecularly templated materials in chemical sensing" ANALYTICA CHIMICA ACTA, ELSEVIER, AMSTERDAM, NL, Bd. 594, Nr. 2, 2. Juli 2007 (2007-07-02), Seiten 147-161, XP022233456 ISSN: 0003-2670
- LIN H Y ET AL: "The microcontact imprinting of proteins: The effect of cross-linking monomers for lysozyme, ribonuclease A and myoglobin" BIOSENSORS & BIOELECTRONICS, ELSEVIER SCIENCE PUBLISHERS, BARKING, GB, Bd. 22, Nr. 4, 15. Oktober 2006 (2006-10-15), Seiten 534-543, XP024961579 ISSN: 0956-5663 [gefunden am 2006-10-15]
- LIN ET AL: "Optimizing the formulation of a myoglobin molecularly imprinted thin-film polymer-formed using a micro-contact imprinting method" BIOSENSORS & BIOELECTRONICS, ELSEVIER SCIENCE PUBLISHERS, BARKING, GB, Bd. 22, Nr. 12, 16. Mai 2007 (2007-05-16), Seiten 3293-3301, XP022080480 ISSN: 0956-5663
- RACHKOV<A> A ET AL: "Towards molecularly imprinted polymers selective to peptides and proteins. The epitope approach" BIOCHIMICA ET BIOPHYSICA ACTA - PROTEIN STRUCTURE AND MOLECULAR ENZYMOLOGIE, ELSEVIER SCIENCE BV, AMSTERDAM, NL, Bd. 1544, Nr. 1-2, 12. Januar 2001 (2001-01-12), Seiten 255-266, XP004279141 ISSN: 0167-4838
- ZHANG HUIQI ET AL: "Non-covalent molecular imprinting with emphasis on its application in separation and drug development", JOURNAL OF MOLECULAR RECOGNITION, vol. 19, no. 4, July 2006 (2006-07), pages 248-259, ISSN: 0952-3499
- RAJKUMAR ET AL: "Development of fructosyl valine binding polymers by covalent imprinting", BIOSENSORS AND BIOELECTRONICS, ELSEVIER BV, NL, vol. 22, no. 12, 16 May 2007 (2007-05-16), pages 3318-3325, XP022080483, ISSN: 0956-5663, DOI: 10.1016/J.BIOS.2007.03.001

## Beschreibung

### Hintergrund der Erfindung

Reagenzien mit der Kapazität zur spezifischen Bindung von Substanzen durch nicht-kovalente Interaktionsformen (Affinitätsbinder) finden vielfältigen Einsatz bei der Erkennung von Biomolekülen und nicht-biologischen Substanzen. In der Medizin bilden solche Reagenzien die Basis moderner diagnostischer Verfahren, in der Pharmazie werden sie als Wirkstoffe eingesetzt. Affinitätsbinder sind weiterhin Grundlage vieler analytischer Methoden, insbesondere in der biomedizinischen Forschung, sowie Trenn- und Reinigungsverfahren in technischen Prozessen.

In biologischen Systemen bildet die affine Erkennung und Bindung die Grundlage zahlreicher zellulärer Prozesse. Beispiele sind Protein-Protein-Interaktionen im Rahmen von Signalwegen und regulatorischen Vorgängen oder die Bindung und Neutralisierung von Erregern, Toxinen oder anderen Fremdstoffen durch spezifische Antikörper infolge der Immunantwort. Antikörper sind eine hochvariable Proteinklasse. Im Verlauf der Immunreaktion können Antikörper auf natürlichem Wege im Prinzip gegen beliebige organismusfremde Substanzen entwickelt und selektiert werden. Die Variabilität beruht auf einem Mechanismus, der zu einer zufälligen Kombination von Aminosäuren in der Antikörperbindungsdomäne - dem Paratop - führt. Auf diese Weise entstehen Binder einzigartiger Spezifität und Affinität. Antikörper, die spezifisch mit einer bestimmten Substanz reagieren, lassen sich darüber hinaus gezielt erzeugen, indem ein (Wirts-)Organismus mit dieser Substanz methodisch in Kontakt gebracht (immunisiert) wird. In speziellen Verfahren können auf diese Weise Antikörpermoleküle einer singulären Struktur und Spezifität erzeugt werden, sogenannte monoklonale Antikörper (Köhler und Milstein 1975, 1984 Nobelpreis für Medizin).

Nicht-kovalente Bindungen zwischen Substanzen basieren auf physikochemischen Interaktionsformen, z. B. Wasserstoffbrückenbindungen, ionischen und hydrophoben Wechselwirkungen und van-der-Waals Kräften, sowie deren räumlicher Anordnung und Passfähigkeit (induced fit).

Speziell bei Protein-Protein-Interaktionen, beispielsweise der Bindung zwischen einem Antikörper und einem Proteinantigen, sind die physikochemischen Wechselwirkungen häufig durch kurze, kontinuierliche Aminosäureabfolgen innerhalb des Moleküls (Peptide) determiniert. Dies gilt sowohl für die Bindungsdomäne des Antikörpers (Paratop, z. B. Laune et al. 2002), als auch für diejenige des Proteins (Epitop, z. B. Andresen et al. 2006). Aus diesem Grund lassen sich bei diesen Formen der molekularen Wechselwirkungen die Antikörper und Proteine durch funktionelle peptidische Binder substituieren. Diese weisen häufig die gleichen oder nur leicht veränderte Bindungseigenschaften hinsichtlich Spezifität und Affinität wie die ursprünglichen Moleküle auf.

Andere biochemische Interaktionsformen lassen sich wiederum auf die physikochemische Wechselwirkung von Kohlenhydraten mit anderen Molekülen (Proteinen, Peptiden, Kohlenhydraten) zurückführen. Alternativ können spezifische Bindermoleküle artifiziell erzeugt werden, beispielsweise als Peptid-Mimetika oder Nukleinsäureaptamere (Kleinjung et al. 1998).

Eine Kategorie vollsynthetischer Reagenzien zur Bindung von Molekülen stellen die molekular geprägten Polymere (MIPs) dar. Molekulare Prägung, auch als Templatverfahren bezeichnet, beinhaltet das Anordnen von polymerisierbaren funktionellen Monomeren um ein Druckmolekül (engl. "print molecule"). Dies wird durch die Anwendung von nicht kovalenten Wechselwirkungen oder durch reversible kovalente Wechselwirkungen (kovalente Prägung) zwischen dem Druckmolekül und den funktionellen Monomeren erreicht. Die so erhaltenen Komplexe werden dann durch Polymerisation in eine hoch vernetzte, makroporöse Polymermatrix eingebracht. Eine Extraktion des Druckmoleküls lässt im Polymer Stellen mit spezieller Gestalt und funktionellen Gruppen, die komplementär zum ursprünglichen Druckmolekül sind, zurück (Mosbach et al. 1994, Wulff et al. 1993).

Molekular geprägte Polymere zur Bindung eines Zielmoleküls werden beispielsweise von Chou et al. in Anal. Chim. Acta., Bd. 542, Nr. 1, 20-25 (2005), Holthoff et al. in Anal. Chim. Acta., Bd. 594, Nr. 2, 147-61 (2007) und Zhang et al. in J. Molec. Recognition, Bd. 19. Nr. 4, 248-259 (2006) beschrieben.

Keine dieser Druckschriften offenbart jedoch die Verwendung von zielspezifischen Peptidliganden in solchen molekular geprägten Polymeren.

Die im Stand der Technik bekannten Reagenzien zur Bindung von Substanzen (z. B. Antikörper, Proteine, Peptide, MIPs) weisen trotz der oben genannten Bedeutung immer noch gewisse Nachteile auf.

Die Herstellung von Antikörpern ist zwangsläufig mit Tierversuchen verbunden, da die Tiere als Wirtsorganismen zur Antikörperbildung und -produktion benötigt werden. In speziellen Fällen können zudem keine Antikörper generiert werden, da die Substanzen entweder toxisch sind und zum Tod des Tieres führen würden, oder da die Substanzen zu schnell im Organismus abgebaut werden, sodass keine wirksame Immunreaktion generiert werden kann. Die Herstellung von Antikörpern im Allgemeinen und monoklonalen Antikörpern im Speziellen, sowie die Herstellung von Proteinen ist darüber hinaus mit hohem Zeit- und Kostenaufwand verbunden. Proteine und Antikörper sind chemisch und physikalisch empfindlich, so dass unter ungünstigen Milieubedingungen (z. B. Temperatur, pH-Wert, Feuchte, chemische Agenzien) ein Verlust der Bindungskapazität eintreten kann.

Peptidische Binder können ebenso wie einfache Kohlenhydrate oder Nukleinsäureaptamere synthetisch in großen Mengen kostengünstig hergestellt werden und sind im Vergleich zu Proteinen/Antikörpern chemisch und physikalisch relativ stabil. Nachteilig ist bei diesen Bindern häufig die unzureichende Stärke der Bindung an die Zielsubstanz aufgrund einer fehlenden räumlichen Struktur des Binders, die den Bindungsvorgang unterstützen kann.

Molekular geprägte Polymere (MIPs) sind chemisch und physikalisch stabil und können synthetisch und daher kostengünstig hergestellt werden. Dem Bindungsvorgang der MIPs liegt in erster Linie die räumliche Passfähigkeit der Zielsubstanz in die Bindungstasche des Polymers zu Grunde. Aus diesem Grund fehlt diesen Bindern häufig die Spezifität und Affinität der physikochemischen Interaktionsformen von biomolekularen Komponenten.

Der vorliegenden Erfindung liegt daher die Aufgabe zu Grunde, neue verbesserte Reagenzien mit der Kapazität zur Erkennung und Bindung von Substanzen durch nicht-kovalente Interaktionsformen (Affinitätsbinder) bereitzustellen. Solche Affinitätsbinder bieten breite Einsatzmöglichkeiten in der Medizin und Diagnostik, Analytik und Bioanalytik, Pharmazie, in Separations- und Reinigungsverfahren und können in diesen Bereichen die Herstellung und Handhabung erleichtern sowie die Kosteneffizienz, Vielseitigkeit, Stabilität, Spezifität und Sensitivität von herkömmlichen Verfahren erhöhen. Eine damit verbundene Aufgabe ist die Bereitstellung eines besonders vorteilhaften Verfahrens zur Herstellung solcher Affinitätsbinder.

Diese Aufgaben werden erfindungsgemäß gelöst durch die Bereitstellung der Verfahren zur Herstellung dieser Affinitätsbinder nach den Ansprüchen 1-6, der Affinitätsbinder mit den Merkmalen der Ansprüche 7-14 und deren Verwendung nach Anspruch 15.

### Beschreibung der Erfindung

Das erfindungsgemäße Verfahren zur Herstellung von Affinitätsbindern mit affinen oder hochaffinen Bindestrukturen für nicht-kovalente Interaktionsformen umfasst die folgenden Schritte:
a) Kontaktieren einer Zielsubstanz mit mehreren Peptidliganden, die zur nicht-kovalenten Bindung an die Zielsubstanz in der Lage sind, für einen ausreichenden Zeitraum, dass sich die Peptidliganden spezifisch an die Zielregionen der Zielsubstanz anlagern können und einen Peptidligand-Zielsubstanz-Komplex bilden, wobei die Peptidliganden eine reaktionsfähige Gruppierung aufweisen, welche mit einem anorganischen und/oder organischen Quervernetzer eine Vernetzungsreaktion eingehen kann;
b) Einbetten des Komplexes in eine strukturgebende Komponente und Fixierung über eine Bindung der Peptidliganden des Komplexes an die strukturgebende Komponente, wobei die Fixierung eine Vernetzungsreaktion der Peptidliganden mit einem organischen und/oder anorganischen Quervernetzer beinhaltet, und wobei die strukturgebende Komponente ein organisches oder anorganisches Polymer umfasst, welches durch eine Polymerisationsreaktion zwischen den reaktionsfähigen Gruppierungen und einem oder mehreren Quervernetzern sowie gegebenenfalls zwischen den Quervernetzern untereinander entsteht;
c) Entfernen der Zielsubstanz, sodass eine Struktur mit definierter räumlicher Anordnung der Peptidliganden entsteht, welche die Kapazität besitzt, die betreffende Zielsubstanz wieder spezifisch zu binden.

Die Peptidliganden können identisch sein und mit wiederkehrenden Strukturmerkmalen (Motiven) der Zielsubstanz interagieren. Alternativ können die Liganden auch unterschiedlich sein und mit verschiedenen Motiven der Zielsubstanz interagieren.

Ein erfindungsgemäßer Affinitätsbinder, der mit dem obigen Verfahren erhältlich ist, umfasst eine strukturgebende Komponente, beispielsweise ein durch eine bestimmte Zielsubstanz molekular geprägtes Polymer, in das affine Liganden für die Zielsubstanz integriert sind.

Als Liganden werden hier Moleküle bezeichnet, die durch nicht-kovalente Interaktionsformen, z.B. Wasserstoffbrückenbindungen, ionische/hydrophobe Wechselwirkungen und van-der-Waals Kräfte sowie "induced fit", die Kapazität zur spezifischen Interaktion (d. h. selektiv und mit messbarer Bindungsstärke) mit derselben Zielsubstanz besitzen. Die erfindungsgemäß verwendeten Liganden sind Peptide.

Mithilfe bioinformatischer, kombinatorischer und/oder molekularbiologischer Verfahren lassen sich erfindungsgemäße Affinitätsbinder für nahezu beliebige Zielsubstanzen generieren.

Typischerweise ist die Zielsubstanz aus der Gruppe ausgewählt, die ein Protein, Peptid, Kohlenhydrat, insbesondere ein (Oligo)saccharid, Lektin, eine Nukleinsäure, oder eine andere makromolekulare Einheit, einschließlich einer prokaryotischen oder eukaryotischen Zelle, eines Virus oder Komponenten davon, umfasst, ist jedoch nicht darauf beschränkt.

In einer Ausführungsform ist die Zielsubstanz ein Mitglied eines bekannten spezifischen Bindungspaares bzw. einer Klasse spezifischer Bindungspaare. Einige typische nicht-beschränkende Beispiele hierfür sind Biotin/Avidin, Biotin/- Streptavidin, Antikörper/Antigen, Rezeptor/Ligand, Lektin/- Saccharid, DNA/DNA, RNA/RNA, DNA/RNA etc. Weitere geeignete Beispiele sind dem Fachmann bekannt oder unschwer in der Literatur zu finden.

Die erfindungsgemäßen Affinitätsbinder erfordern die Bereitstellung geeigneter Liganden für die jeweilige Zielsubstanz.

Die Liganden können beispielsweise aus den bekannten Liganden für eine bestimmte Zielsubstanz ausgewählt sein und somit das zweite Mitglied eines bekannten spezifischen Bindungspaares darstellen.

Einige nicht-beschränkende Beispiele für geeignete Peptidliganden sind bindende Antikörperfragmente gegen ein oder mehrere Zielproteine, z.B. native oder gentechnisch modifizierte Fab-Fragmente oder einkettige Fab-Derivate zur Affinitätsbindung des entsprechenden Antigens, z.B. Tumornekrosefaktor-Alpha, Peptide aus dem Paratop eines Antikörpers, z.B. gegen Angiotensin II, Lysozym etc., Fab-Fragmente zur Affinitätsbindung von Viruspartikeln etc. Andere allgemeinere Beispiele sind monovalente oder polyvalente Aptamere zur Bindung von Proteinen, Partikeln, Zellen oder niedermolekularen Stoffen (z.B. Citrullin, Flavinmononucleotid, Neomycin B etc.).

Eine Vielfalt solcher Liganden gegen verschiedene Zielsubstanzen ist bereits im Handel erhältlich und verwendbar.

Es ist jedoch auch möglich, neue Liganden für eine Zielsubstanz zu entwickeln und zu selektionieren. Die Liganden können durch molekularbiologische Verfahren aus anderen bindungsbefähigten Molekülen (z.B. Proteine, Antikörper) abgeleitet oder auch de *novo* generiert werden. Alternativ können sie durch Kartierungsverfahren (z.B. Epitopmapping, Paratopmapping) bekannter Strukturen, in kombinatorischen Verfahren oder durch computergestützte Modellierungsverfahren bestimmt werden.

Die Selektion kann im Fall peptidischer Liganden beispielsweise erfolgen durch die molekularbiologische Untersuchung und Charakterisierung des Paratops von monoklonalen Antikörpern oder des Epitops von Proteinen, die in der Folge auf biologischem oder synthetischem Weg nachgebildet werden. Alternativ können peptidische Liganden durch Techniken des Phagendisplays selektiert und hergestellt werden.

Eine weitere Alternative für die Selektion von peptidischen Liganden sind insbesondere Methoden des Epitop-Mappings oder des Paratop-Mappings. Diese Verfahren können auf bekannten Sequenzinformationen der betreffenden Proteine bzw. Antikörper beruhen, aber auch auf der Grundlage kombinatorischer Peptidbibliotheken erfolgen. Weiterhin kann die Selektion der peptidischen Liganden durch computergenerierte Vorhersagen und erfolgen oder durch diese unterstützt werden.

Die identifizierten Liganden werden anschließend vorzugsweise synthetisch hergestellt und im Verlauf der Synthese mit einer chemischen Funktion (Linker) ausgestattet, die im Verlauf der Herstellung der Affinitätsbinder die Integration und Vernetzung mit der strukturgebenden Komponente ermöglicht. Auch bekannte und z.B. im Handel bezogene Liganden können erforderlichenfalls nachträglich mit gewünschten Linkerfunktionen versehen werden. Bei Peptiden und Proteinen und anderen organischen Molekülen können hierfür bekannte Biokonjugationsprotokolle unter Nutzung von beispielsweise freien Thiol-, Carboxy- oder Aminofunktionen verwendet werden.

Die Linkerfunktion kann grundsätzlich jede bindungsfähige Gruppierung sein, die eine Fixierung der Liganden in der strukturgebenden Komponente gestattet. Typischerweise handelt es sich um eine reaktionsfähige Gruppierung, die mit einem anorganischen und/oder organischen Quervernetzer eine Vernetzungsreaktion eingehen kann. Bei dieser Vernetzungsreaktion handelt es sich vorzugsweise um eine Polymerisationsreaktion, wobei der Begriff "Polymerisation" hier auch eine Polykondensation oder Polyaddition einschließen soll, und sowohl die Linkerfunktion als auch der/die Quervernetzer tragen polymerisierbare Gruppen.

Ein anorganischer Quervernetzer kann beispielsweise ein bifunktionelles oder multifunktionelles Organosiloxan sein.

Im Falle eines organischen Quervernetzers kann es sich beispielweise um ein Acrylderivat, Methacrylderivat, z.B. Ethylenglycoldimethacrylat oder Trimethylolpropantrimethacrylat, oder ein Allylderivat handeln. Um eine größere Hydrophilie des Polymerrückgrats zu erreichen, können z.B. entsprechende, auf Polyethylenglycol basierende Quervernetzer eingesetzt werden. Weitere geeignete Beispiele für Quervernetzer werden für den Fachmann unschwer ersichtlich sein.

In einer Ausführungsform findet die Polymerisationsreaktion nur zwischen den Linkergruppierungen und einem oder mehreren Quervernetzern sowie gegebenenfalls zwischen den Quervernetzermolekülen untereinander statt. Die Polymerisationsreaktion kann jedoch auch in Gegenwart weiterer polymerisationsfähiger monomerer, oligomerer oder polymerer Reaktionspartner, die mit den Linkergruppierungen und/oder dem Quervernetzer eine Polymerisationsreaktion eingehen können, stattfinden.

Geeignete Reaktionspartner für die jeweiligen Linkerfunktionen bzw. Quervernetzer sind dem Fachmann bekannt oder unschwer durch Routineversuche zu ermitteln. Die Reaktionspartner können z.B. auch andere polymerisierbare Affinitätsbinder sein. Die Polymerisationsreaktion kann z.B. eine RAFT-Polymerisation sein.

Durch die Polymerisationsreaktion entsteht eine strukturgebende Komponente, die (neben den integrierten Liganden) ein organisches oder anorganisches Polymer, z.B. ein Polyurethan, Polyharnstoff, Polyester, Polyamid, Aminoplast, Epoxidharz, Silikone, oder deren Copolymere oder Mischungen, umfassen kann oder im Wesentlichen daraus besteht.

Beispiele für geeignete Linkerfunktionen sind z.B. ein Acrylderivat, Methacrylderivat wie Ethylenglycoldimethacrylat oder Trimethylolpropantrimethacrylat, Epoxide, Isocyanate oder ein Allylderivat.

Die Vernetzungsreaktion, insbesondere Polymerisation, kann radikalisch, z. B. lichtinduziert oder thermisch induziert bei ausreichend niedrigen Temperaturen, um die Zielsubstanz nicht zu beeinträchtigen, anionisch oder kationisch ablaufen.

Bei der Herstellung der Affinitätsbinder wird eine Matrize der Zielsubstanz mit den Liganden versetzt, so dass sich die Liganden spezifisch an die Zielregionen der Zielsubstanz anlagern können. Im nächsten Schritt werden diese Komplexe in die strukturgebende Komponente gebettet und dort fixiert, vorzugsweise mit dieser Komponente wie oben erläutert vernetzt. Anschließend wird die Zielsubstanz wieder entfernt, sodass eine Struktur mit definierter räumlicher Anordnung der Liganden entsteht.

Dieses Produkt besitzt die Kapazität, die betreffende Zielsubstanz wieder spezifisch zu binden, d.h. der nun vorhandene erfindungsgemäße Affinitätsbinder besitzt die Kapazität, die Zielsubstanz selektiv und mit definierter Stärke wieder zu binden.

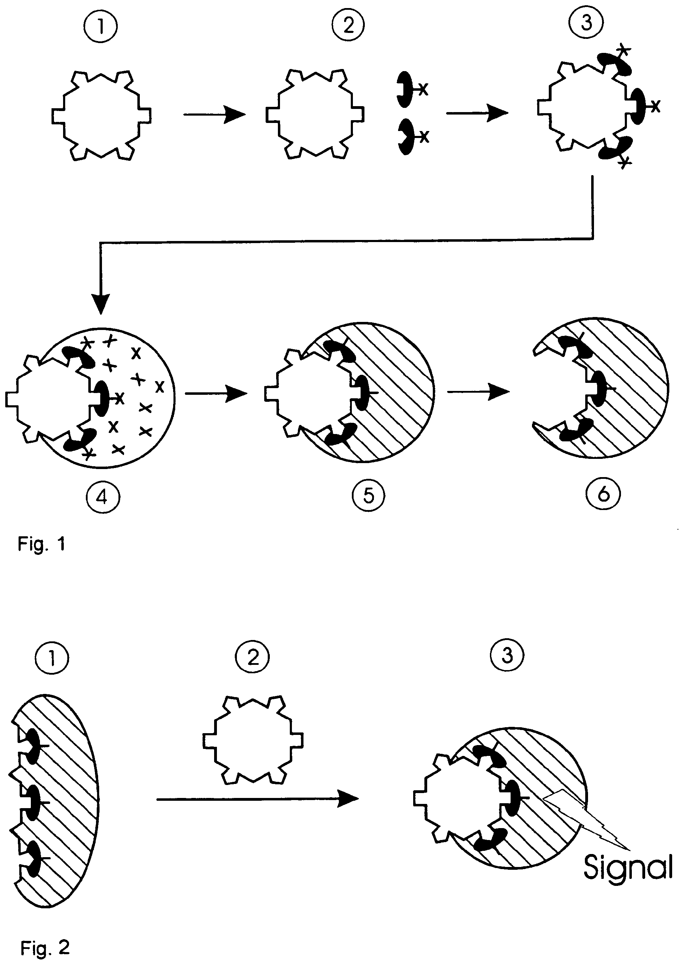

In einer alternativen Ausführungsform handelt es sich bei dem Affinitätsbinder nicht wie in Fig. 1 und 2 dargestellt um einen einzelnen Körper mit einer Bindungstasche, sondern um eine dreidimensionale. Matrix mit einer Vielzahl von Bindungsstellen.

In einer besonders vorteilhaften Ausführungsform der Affinitätsbinder sind diese mit einer Effektorfunktion ausgestattet, sodass der Bindungsvorgang der Zielsubstanz in ein sichtbares oder messbares oder in durch andere Formen charakterisiertes Signal übertragen wird (Fig 2). Auslöser für die Effektorfunktion können beispielsweise Konformationsänderungen, Striktion, Ladungsübertragung, Reassoziation, Energiedissipation oder ähnliche Mechanismen sein. Die Effektorfunktion kann auf Lichtaktivität (Fluoreszenz, Phosphoreszenz, Chemilumineszenz), Farbänderung, Morphologieänderung, Elektronenübertragung, Ladungsseparation und Entladung oder anderen Prinzipien beruhen.

Ein erfindungsgemäßer Affinitätsbinder, der mit dem obigen Verfahren erhältlich ist, umfasst eine strukturgebende Komponente, beispielsweise ein durch eine bestimmte Zielsubstanz molekular geprägtes Polymer, in das mehrere affine Liganden für die Zielsubstanz integriert sind.

Die Verwendung der erfindungsgemäßen Affinitätsbinder bietet folgende Vorteile:
Durch die Kombination der strukturgebenden Komponente, die eine räumliche Passfähigkeit der Zielsubstanz (induced fit) gewährleistet und gleichzeitig die räumliche Anordnung der integrierten Liganden unterstützt, und der nicht-kovalenten molekularen Interaktion der Zielsubstanz mit den Liganden wird eine effektive Bindungsstärke erreicht, die zu einer deutlichen Erhöhung der Bindungsstärke gegenüber den jeweiligen Einzelkomponenten führt.

Die Bindungsstärke erhöht sich exponentiell, wenn statt eines Liganden mehrere Liganden an definierten Positionen der strukturgebenden Komponente integriert werden, die in der Folge mit verschiedenen Domänen der Zielsubstanz interagieren können. Dabei können die Liganden identisch sein und mit einem wiederkehrenden Strukturmerkmal der Zielsubstanz reagieren, aber es kann sich auch um unterschiedliche Liganden handeln, die mit nur einem bestimmten (singulären) Strukturmerkmal der Zielsubstanz reagieren.

Durch das Zusammenwirken mehrerer Liganden kann eine stufenweise und rational regulierbare Bindungsstärke (Avidität) erreicht werden, die mit herkömmlichen und insbesondere natürlichen Affinitätsbindern nicht realisierbar ist. Die Bindungsstärke kann so gewählt werden, dass eine zerstörungsfreie Rückgewinnung der Zielsubstanz möglich ist (z. B. für Separations-/Reinigungsverfahren), aber auch irreversibel gestaltet werden (z. B. für analytische Verfahren mit dem Ziel höchster Sensitivität).

Sowohl die strukturgebenden Komponenten, als auch die Liganden lassen sich vollsynthetisch herstellen. Aus diesem Grund sind die erfindungsgemäßen Affinitätsbinder schneller und kostengünstiger herzustellen als proteinische Binder (z.B. Antikörper).

Die erfindungsgemäßen Affinitätsbinder sind chemisch und physikalisch deutlich stabiler als proteinische Binder und können daher unter extremen Milieubedingungen eingesetzt werden.

Die Entwicklung und Herstellung der erfindungsgemäßen Affinitätsbinder ist prinzipiell unabhängig von Tierversuchen und/oder gentechnischen Verfahren zu gestalten.

Die erfindungsgemäßen Affinitätsbinder bieten breite Einsatzmöglichkeiten, beispielsweise in (aber nicht beschränkt auf) Medizin und Diagnostik, Analytik und Bioanalytik, Pharmazie, Separations- und Reinigungsverfahren, und können in diesen Bereichen die Herstellung und Handhabung erleichtern und/oder die Kosteneffizienz, Vielseitigkeit, Stabilität, Spezifität und Sensitivität von herkömmlichen Verfahren erhöhen.

### Figurenbeschreibung

**Fig. 1****:** Schema der Generierung erfindungsgemäßer Affinitätsbinder durch die Kombination von Liganden und einer strukturgebenden Komponente. 1: Zielsubstanz; 2: Zugabe eines oder mehrerer Liganden, die mit einer polymerisierbaren Funktion (X) ausgestattet sind; 3: Anlagerung der Liganden an die Zielsubstanz; 4: Einbettung der Komplexe aus Zielsubstanz und Peptiden in die strukturgebende Komponente (Polymerprodukt von X); 5: Vernetzung der Bestandteile unter Entstehung einer räumlichen Struktur, in die die Liganden fest und an definierten Positionen integriert sind; 6: Entfernung der Matrize der Zielsubstanz aus der Struktur.
**Fig. 2****:** Schema des erfindungsgemäßen Affinitätsbinders, bei dem der Bindungsvorgang mit einer Effektorfunktion kombiniert ist. 1: Erfindungsgemäßer Affinitätsbinder im bindungsbefähigten Ausgangszustand; 2: Spezifische Zielsubstanz; 3: Bindungsvorgang der Zielsubstanz führt im erfindungsgemäßen Affinitätsbinder zu Veränderungen, die eine Effektorfunktion darstellen oder diese auslösen.

Die folgenden Ausführungsbeispiele sollen die vorliegende Erfindung näher erläutern, ohne diese jedoch auf die Details dieser Beispiele zu beschränken.

Das Konzept der vorliegenden Erfindung lässt sich beispielsweise zur Herstellung eines Affinitätsbinders (einer Affinitätsstruktur) für die Bindung des hochmolekularen Proteins Thyreoglobulin anwenden. Thyreoglobulin wird in der klinischen Chemie als Tumormarker für Schilddrüsenkarzinome herangezogen. Der besonders empfindliche Nachweis von Tumormarkern kann eine frühere Diagnose von Tumorgeschehen ermöglichen und die Heilungschancen verbessern. Die Affinitätsstruktur kann daher als Grundlage eines Testsystems für den verbesserten Nachweis von Thyreoglobulin eingesetzt werden.

### BEISPIEL 1

### Identifizierung und Herstellung von Liganden für Thyreoglobulin

### 1. Paratopkartierung des monoklonalen anti-Thyreoglobulin Antikörpers mAk Tg10 (Vorgehensweise analog Daniel Laune u. a. 2002)

Genetische Analyse und Nukleotidsequenzierung der VH und VL Regionen: Die Gene der leichten und der schweren Kette des Immunglobulin G der Hybridomzelllinie Tg10 wurden molekularbiologisch unter Einsatz der Polymerasekettenreaktion und Primerpaaren, die in den Anfangsbereichen der C_{H}1- und C_{L}-Regionen und in konservierten Bereichen der Leaderpeptidsequenz der Maus-Ig Genfamilie binden, amplifiziert (Chardes u. a., 1999). Die Nukleotidsequenz der Amplifikate wurde anschließend sequenziert und in die entsprechenden Aminosäureabfolgen übersetzt.

Paratopkartierung: Die Aminosäuresequenz der oben identifizierten V_{H}- und V_{L}-Domänen wurde durch überlappende Peptide je 12 Aminosäuren mit einem Versatz von je einer Aminosäure substituiert. Die Peptide wurden synthetisch hergestellt und in einem Arrayformat immobilisiert. Die Kapazität der Peptide zur Bindung des Thyreoglobulins wurde mithilfe eines markierten Antigens in Konzentrationen zwischen 10⁻⁷ und 10⁻⁹ M getestet. Das Peptid (A) mit der Aminosäuresequenz Ac-FTFGSG aus der komplementaritätsbestimmenden Region (CDR) L3 und das Peptid (B) mit der Aminosäuresequenz TFTSYGLTWVIQ aus der CDR H1 des Tg10 wurden als geeignete Liganden identifiziert.

### BEISPIEL 2

### Herstellung eines Affinitätsbinders für Human-Thyreoglobulin

Die Herstellung des Affinitätsbinders (der Affinitätsstrukturen) erfolgte durch molekulares Prägen eines Polymers unter Integration der peptidischen Liganden.

Die in Beispiel 1 identifizierten Peptide A und B wurden synthetisch hergestellt und im Verlauf der Synthese mit einem Abstandshaltermolekül (Spacer) (z.B. einer Alkyl- oder Polyethereinheit) und einer polymerisierbaren Gruppe (Linker) (z.B. ein Acrylderivat oder Methacrylderivat) ausgestattet. Die Peptidsynthese erfolgte an der Festphase nach Fmoc-Strategie (Merrifield 1963, Carpino und Hahn 1970). Humanes Thyreoglobulin wird im jeweiligen erforderlichen Verhältnis mit jeweils einem der Peptide in Pufferlösungen oder Pufferlösungen gemischt mit organischen Lösungsmitteln (MeOH, Acetonitril) versetzt. Die Komplexe aus Thyreoglobulin und Peptid werden in polymerisierbarem Quervernetzer gelöst/- suspendiert. Die Quervernetzer können z.B. Ethylenglycoldimethacrylat oder das dreifachfunktionelle Vernetzungsmittel TRIM (Trimethylolpropantrimethacrylat) sein. Falls eine größere Hydrophilie des Polymerrückgrats erwünscht ist, werden entsprechende polyethylenglycol-basierte Quervernetzer eingesetzt. Anschließend erfolgt die Vernetzung zum Polymer lichtinduziert oder thermisch bei relativ tiefen Temperaturen, die das Protein nicht beinträchtigen. Das als Matrize verwendete Thyreoglobulin wird aus dem Polymer entfernt, sodass ein molekular geprägtes Polymer mit integriertem Peptidliganden erhalten wird.

Die auf diese Weise hergestellten Affinitätsstrukturen sind geeignet, Thyreoglobulin aus einer Probenlösung selektiv zu binden und sensitiv nachzuweisen.

### BEISPIEL 3

### Herstellung eines Affinitätsbinders für Anti-Tumornekrosefaktor-Alpha

Im Handel erhältlicher Anti-Tumornekrosefaktor-Alpha-Antikörper (Santa Cruz Biotechnology Inc., Santa Cruz, CA, USA) wird proteolytisch in Fab-Fragmente zerlegt ("Fab Preparation Kit", Pierce Biotechnology, Rockford, IL, USA), welche in der Lage sind, Tumornekrosefaktor-Alpha spezifisch zu binden. Die Fab-Fragmente wurden über ein EDC/NHS-Biokonjugationsprotokoll (Hermanson 1996) mit einem polymerisierbaren Linker versehen und dann mit der Zielsubstanz Tumornekrosefaktor-Alpha in Kontakt gebracht. Die Komplexe aus Fab-Fragmenten und der Zielsubstanz werden in polymerisierbarem Quervernetzer (z.B. Ethylenglycoldimethacrylat oder TRIM) gelöst/suspendiert und analog zu Beispiel 2 polymerisiert. Der als Matrize verwendete Tumornekrosefaktor-Alpha wird aus dem Polymer entfernt, sodass ein molekular geprägtes Polymer mit integrierten Fab-Fragmenten erhalten wird.

Die auf diese Weise hergestellten Affinitätsstrukturen sind geeignet, Tumornekrosefaktor-Alpha aus einer Probenlösung selektiv zu binden und sensitiv nachzuweisen.

### Literatur

Andresen, H., Zarse, K., Grötzinger, C., Hollidt, J., Ehrentreich-Förster, E., Bier, F. F., Kreuzer, O. J.: Development of peptide microarrays for epitope mapping of antibodies against the human TSH receptor. J Immunol Methods 315 (2006), 11-18.
Chardes, T., Villard, S., Ferrieres, G., Piechaczyk, M., Cerutti, M., Devauchelle, G., Pau, B. : Efficient amplification and direct sequencing of mouse variable regions from any immunoglobulin gene family. FEBS Lett 452 (1999), 386.
Conrad, R. C., Baskerville, S., Ellington, A. D.: In vitro selection methodologies to probe RNA function and structure. Mol Divers 1 (1995), 69-78.
Kleinjung, F., Klußmann, S., Erdmann, V.A., Scheller, F.W., Fürste, J.P., Bier, F.F.: High Affinity RNA as a Recognition Element in a Biosensor. Anal Chem 70 (1998), 328-331.
Köhler, G. & Milstein, C.: Continuous cultures of fused cells secreting antibody of predefined specificity. Nature 256 (1975), 495-497.
Laune, D., Molina, F., Ferrieres, G., Villard, S., Bes, C., Rieunier, F., Chardes, T., Granier, C.: Application of the Spot method to the identification of peptides and amino acids from the antibody paratope that contribute to antigen binding. J Immunol Methods 267 (2002), 53-70.
Mosbach, K.: Molecular imprinting. Trends Biochem Sci 19 (1994), 9-14
Wulff, G.: The role of binding-site interactions in the molecular imprinting of polymers. Trends Biotechnol 11 (1993), 85-87

## Patentansprüche

1. Verfahren zur Herstellung von Affinitätsbindern mit affinen oder hochaffinen Bindestrukturen für nicht-kovalente Interaktionsformen, umfassend
a) Kontaktieren einer Zielsubstanz mit mehreren Peptidliganden, die zur nicht-kovalenten Bindung an die Zielsubstanz in der Lage sind, für einen ausreichenden Zeitraum, dass sich die Peptidliganden spezifisch an die Zielregionen der Zielsubstanz anlagern können und einen Peptidligand-Zielsubstanz-Komplex bilden, wobei die Peptidliganden eine reaktionsfähige Gruppierung aufweisen, welche mit einem anorganischen und/oder organischen Quervernetzer eine Vernetzungsreaktion eingehen kann;
b) Einbetten des Komplexes in eine strukturgebende Komponente und Fixierung über eine Bindung der Peptidliganden des Komplexes an die strukturgebende Komponente, wobei die Fixierung eine Vernetzungsreaktion der Peptidliganden mit einem organischen und/oder anorganischen Quervernetzer beinhaltet, und wobei die strukturgebende Komponente ein organisches oder anorganisches Polymer umfasst, welches durch eine Polymerisationsreaktion zwischen den reaktionsfähigen Gruppierungen und einem oder mehreren Quervernetzern sowie gegebenenfalls zwischen den Quervernetzern untereinander entsteht;
c) Entfernen der Zielsubstanz, sodass eine Struktur mit definierter räumlicher Anordnung der Peptidliganden entsteht, welche die Kapazität besitzt, die betreffende Zielsubstanz wieder spezifisch zu binden.

2. Verfahren nach Anspruch 1, **dadurch gekennzeichnet, dass** die Liganden
a) identisch sind und mit wiederkehrenden Motiven der Zielsubstanz interagieren oder
b) unterschiedlich sind und mit verschiedenen Motiven der Zielsubstanz interagieren.

3. Verfahren nach Anspruch 1 oder 2, **dadurch gekennzeichnet, dass** die Zielsubstanz aus der Gruppe ausgewählt ist, die ein Protein, Peptid, Kohlenhydrat, Lektin, eine Nukleinsäure, oder eine andere makromolekulare Einheit, einschließlich einer prokaryotischen oder eukaryotischen Zelle, eines Virus oder Komponenten davon, umfasst.

4. Verfahren nach einem der Ansprüche 1-3, **dadurch gekennzeichnet, dass** die strukturgebende Komponente ein organisches Polymer ist oder umfasst.

5. Verfahren nach einen der Ansprüche 1-4, **dadurch gekennzeichnet, dass** die Liganden mit einem reaktionsfähigen, insbesondere polymerisationsfähigen, Linker versehen sind, wobei der Linker vorzugsweise aus der Gruppe ausgewählt ist, die aus einem Acrylderivat, Methacrylderivat, Epoxid, Isocyanat oder einem Allylderivat besteht.

6. Verfahren nach einem der Ansprüche 1-5, **dadurch gekennzeichnet, dass** der Quervernetzer polymerisierbare Gruppen trägt, z.B. ein Acrylderivat, Methacrylderivat wie Ethylenglycoldimethacrylat oder Trimethylolpropantrimethacrylat, oder ein Allylderivat ist.

7. Affinitätsbinder mit affinen oder hochaffinen Bindestrukturen für nicht-kovalente Interaktionsformen, erhältlich mit dem Verfahren nach einem der Ansprüche 1-6 und umfassend eine strukturgebende Komponente und mehrere Peptidliganden, die an definierten Positionen in die strukturgebende Komponente integriert sind und mit verschiedenen Domänen der Zielsubstanz interagieren können, **dadurch gekennzeichnet, dass** die strukturgebende Komponente die Peptidliganden in einer bestimmten räumlichen Anordnung hält, welche die spezifische Bindung der Peptidliganden an eine Zielsubstanz ermöglicht oder beeinflusst, wobei die strukturgebende Komponente ein organisches oder anorganisches Polymer umfasst, welches durch eine Polymerisationsreaktion zwischen reaktionsfähigen Gruppierungen der Peptidliganden und einem oder mehreren Quervernetzern sowie gegebenenfalls zwischen den Quervernetzern untereinander entstanden ist.

8. Affinitätsbinder nach Anspruch 7, **dadurch gekennzeichnet, dass** die spezifische räumliche Anordnung die Bindung der Liganden an die Zielsubstanz mit einer einstellbaren Bindungsstärke ermöglicht.

9. Affinitätsbinder nach einem der Ansprüche 7-8, **dadurch gekennzeichnet, dass** die Gestalt der strukturgebenden Komponente eine räumliche Einpassung der Zielsubstanz (induced fit) in die strukturgebende Komponente ermöglicht und dadurch die Bindung der Zielsubstanz unterstützen kann.

10. Affinitätsbinder nach einem der Ansprüche 7-9, **dadurch gekennzeichnet, dass**
a) die Liganden identisch sind und mit wiederkehrenden Motiven der Zielsubstanz interagieren oder
b) die Liganden unterschiedlich sind und mit verschiedenen Motiven der Zielsubstanz interagieren.

11. Affinitätsbinder nach einem der Ansprüche 7-10, **dadurch gekennzeichnet, dass** die Zielsubstanz aus der Gruppe ausgewählt ist, die ein Protein, Peptid, Kohlenhydrat, Lektin, eine Nukleinsäure, oder eine andere makromolekulare Einheit, einschließlich einer prokaryotischen oder eukaryotischen Zelle, eines Virus oder Komponenten davon, umfasst.

12. Affinitätsbinder nach einem der Ansprüche 7-11, **dadurch gekennzeichnet, dass** die strukturgebende Komponente ein molekular geprägtes Polymer ist oder umfasst.

13. Affinitätsbinder nach einem der Ansprüche 7-12, **dadurch gekennzeichnet, dass** die Bindung der Zielsubstanz mit einer Effektorfunktion gekoppelt ist.

14. Affinitätsbinder nach Anspruch 13, **dadurch gekennzeichnet, dass** die Effektorfunktion aus der Gruppe ausgewählt ist, die eine Konformationsänderung, eine Striktion, eine Ladungsübertragung, eine Reassoziation, eine Energiedissipation, eine pH-Änderung, eine Leitfähigkeitsänderung und eine katalytische Funktion umfasst.

15. Verwendung des Affinitätsbinders nach einem der Ansprüche 7-14 in der Medizin, insbesondere der Diagnostik, in der Analytik, insbesondere Bioanalytik, in der Pharmazie, in Separations- und Reinigungsverfahren.

## Claims

1. A method for the production of affinity binders having affinitive or highly affinitive binding structures for non-covalent interaction types, comprising
a) contacting a target substance with several peptide ligands which are capable to non-covalently bind to the target substance for a sufficient time that the peptide ligands can specifically attach to the target regions of the target substance and form a peptide ligand-target substance complex, wherein the peptide ligands have a reactive moiety which can undergo a cross-linking reaction with an inorganic and/or organic cross-linker;
b) embedding the complex in a structure-providing component and fixing by means of binding the peptide ligands of the complex to the structure-providing component, wherein the fixing comprises a cross-linking reaction of the peptide ligands with an organic and/or inorganic cross-linker and wherein the structure-providing component comprises an organic or inorganic polymer which results from a polymerisation reaction between the reactive moieties and one or more cross-linkers and optionally between the cross-linker molecules amongst each other;
b) removing the target substance such that a structure with a defined spatial arrangement of the peptide ligands is formed which has the capacity to specifically bind the respective target substance again.

2. The method according to claim 1, **characterized in that**
a) the ligands are identical and interact with recurring motifs of the target substance, or
b) the ligands are different and interact with different motifs of the target substance.

3. The method according to claim 1 or 2, **characterized in that** the target substance is selected from the group comprising a protein, peptide, carbohydrate, lectin, nucleic acid or another macromolecular unit, including a prokaryotic or eukaryotic cell, a virus or components thereof.

4. The method according to one of claims 1-3, **characterized in that** the structure-providing component is or comprises an organic polymer.

5. The method according to one of claims 1-4, **characterized in that** the ligands are provided with a reactive, in particular polymerisable linker, wherein preferably the linker is selected from the group consisting of an acryl derivative, methacryl derivative, epoxide, isocyanate or allyl derivative.

6. The method according to one of claims 1-5, **characterized in that** the cross-linker bears polymerisable groups, e.g. is an acryl derivative, methacryl derivative such as ethylene glycol dimethacrylate or trimethylolpropane trimethacrylate, or allyl derivative.

7. An affinity binder having affinitive or highly affinitive binding structures for non-covalent interaction types, obtainable with the method according to one of claims 1-6 and comprising a structure-providing component and several peptide ligands integrated into the structure-providing component and capable to interact with different domains of the target substance, **characterized in that** the structure-providing component holds the peptide ligands in a certain spatial arrangement which permits or affects the specific binding of the peptide ligands to a target substance, wherein the structure-providing component comprises an organic or inorganic polymer which is the result from a polymerisation reaction between the reactive moieties and one or more cross-linkers and optionally between the cross-linker molecules amongst each other.

8. The affinity binder according to claim 7, **characterized in that** the specific spatial arrangement permits the binding of the ligands to the target substance with an adjustable affinity.

9. The affinity binder according to one of claims 7-8, **characterized in that** the shape of the structure-providing component allows for a spatial induced fit of the target substance in the structure-providing component and thus can support the binding of the target substance.

10. The method according to one of claims 7-9, **characterized in that**
a) the ligands are identical and interact with recurring motifs of the target substance, or
b) the ligands are different and interact with different motifs of the target substance.

11. The affinity binder according to one of claims 7-10, **characterized in that** the target substance is selected from the group comprising a protein, peptide, carbohydrate, lectin, nucleic acid or another macromolecular unit, including a prokaryotic or eukaryotic cell, a virus or components thereof.

12. The affinity binder according to one of claims 7-11, **characterized in that** the structure-providing component is or comprises a molecularly imprinted polymer.

13. The affinity binder according to one of claims 7-12, **characterized in that** the binding of the target substance is coupled with an effector function.

14. The affinity binder according to claim 13, **characterized in that** the effector function is selected from the group comprising a conformational change, a striction, a charge transfer, a reassociation, an energy dissipation, a pH change, a conductivity change or a catalytic function.

15. The use of the affinity binder according to one of claims 7-14 in medicine, in particular diagnostics, in analytics, in particular bioanalytics, in pharmaceutics, in separation and cleaning methods.

## Revendications

1. Procédé servant à fabriquer des liants d'affinité avec des structures de liaison affines ou à affinité élevée pour des formes d'interaction non covalentes, comprenant
a) la mise en contact d'une substance cible avec plusieurs ligands peptidiques, qui sont en mesure, aux fins de la liaison non covalente avec la substance cible, pour un laps de temps suffisant, de s'accumuler de manière spécifique sur les régions cibles de la substance cible et de former un complexe substance cible et ligand peptidique, dans lequel les ligands peptidiques présentent un groupement réactif, qui peut entrer dans une réaction de réticulation avec un agent de réticulation croisée inorganique et/ou organique ;
b) l'incorporation du complexe dans un composant structurant et la fixation par l'intermédiaire d'une liaison des ligands peptidiques du complexe au composant structurant, dans lequel la fixation comporte une réaction de réticulation des ligands peptidiques avec un agent de réticulation croisée organique et/ou inorganique, et dans lequel le composant structurant comprend un polymère organique ou inorganique, qui apparaît du fait d'une réaction de polymérisation entre les groupements réactifs et un ou plusieurs agents de réticulation croisée ainsi qu'éventuellement entre les agents de réticulation croisée entre eux ;
c) l'élimination de la substance cible de sorte qu'une structure avec une disposition spatiale définie des ligands peptidiques apparaisse, laquelle possède la capacité de lier à nouveau de manière spécifique la substance cible concernée.

2. Procédé selon la revendication 1, **caractérisé en ce que** les ligands
a) sont identiques et interagissent avec des motifs récurrents de la substance cible ou
b) sont différents et interagissent avec des motifs différents de la substance cible.

3. Procédé selon la revendication 1 ou 2, **caractérisé en ce que** la substance cible est choisie parmi le groupe, qui comprend une protéine, un peptide, de l'hydrate de carbone, de la lectine, un acide nucléique ou une autre entité macromoléculaire, y compris une cellule procaryote ou eucaryote, un virus ou des composants de ces derniers.

4. Procédé selon l'une quelconque des revendications 1 - 3, **caractérisé en ce que** le composant structurant est ou comprend un polymère organique.

5. Procédé selon l'une quelconque des revendications 1 - 4, **caractérisé en ce que** les ligands sont pourvus d'un chaînon de liaison réactif, en particulier apte à la polymérisation, dans lequel le chaînon de liaison est choisi de préférence parmi le groupe, qui est constitué d'un dérivé acrylique, d'un dérivé méthacrylique, d'un epoxyde, d'un isocyanate ou d'un dérivé allylique.

6. Procédé selon l'une quelconque des revendications 1 - 5, **caractérisé en ce que** l'agent de réticulation croisée supporte des groupes polymérisables, par exemple est un dérivé acrylique, un dérivé méthacrylique tel qu'un méthacrylate d'éthylène glycol ou d'un triméthacrylate de triméthylolpropane, ou un dérivé allylique.

7. Liant d'affinité avec des structures de liaison affines ou à affinité élevée pour des formes d'interaction non covalentes, pouvant être obtenu avec le procédé selon l'une quelconque des revendications 1 - 6 et comprenant un composant structurant et plusieurs ligands peptidiques, qui sont intégrés en des positions définies dans le composant structurant et qui peuvent interagir avec des domaines différents de la substance cible, **caractérisé en ce que** le composant structurant maintient les ligands peptidiques dans une disposition spatiale donnée, qui permet ou influence la liaison spécifique des ligands peptidiques à une substance cible, dans lequel le composant structurant comprend un polymère organique ou inorganique, qui est apparu du fait d'une réaction de polymérisation entre des groupements réactifs des ligands peptidiques et un ou plusieurs agents de réticulation croisée ainsi qu'éventuellement entre les agents de réticulation croisée entre eux.

8. Liant d'affinité selon la revendication 7, **caractérisé en ce que** la disposition spatiale spécifique permet la liaison des ligands à la substance cible avec une intensité de liaison pouvant être réglée.

9. Liant d'affinité selon l'une quelconque des revendications 7 - 8, **caractérisé en ce que** la forme du composant structurant permet une adaptation spatiale de la substance cible (induced fit) dans le composant structurant et peut ce faisant soutenir la liaison de la substance cible.

10. Liant d'affinité selon l'une quelconque des revendications 7 - 9, **caractérisé en ce que**
a) les ligands sont identiques et interagissent avec des motifs récurrents de la substance cible ou
b) les ligands sont différents et interagissent avec des motifs différents de la substance cible.

11. Liant d'affinité selon l'une quelconque des revendications 7 - 10, **caractérisé en ce que** la substance cible est choisie parmi le groupe, qui comprend une protéine, un peptide, de l'hydrate de carbone, de la lectine, un acide nucléique ou une autre entité macromoléculaire, y compris une cellule procaryote ou eucaryote, un virus ou des composants de ces derniers.

12. Liant d'affinité selon l'une quelconque des revendications 7 - 11, **caractérisé en ce que** le composant structurant est ou comprend un polymère moléculairement imprimé.

13. Liant d'affinité selon l'une quelconque des revendications 7 - 12, **caractérisé en ce que** la liaison de la substance cible est couplée à une fonction effectrice.

14. Liant d'affinité selon la revendication 13, **caractérisé en ce que** la fonction effectrice est choisie parmi le groupe, qui comprend un changement de conformation, une striction, un transfert de charge, une réassociation, une dissipation d'énergie, un changement de pH, un changement de conductivité et une fonction catalytique.

15. Utilisation d'un liant d'affinité selon l'une quelconque des revendications 7 - 14 dans le secteur de la médecine, en particulier du diagnostic, de l'analyse, en particulier de la bioanalyse, de la pharmacie, dans des procédés de séparation et de nettoyage.
